# EUROPÄISCHE PATENTSCHRIFT

(11) **EP 1 776 154 B1**
(45) Veröffentlichungstag und Bekanntmachung des Hinweises auf die Patenterteilung: **23.05.2018**
(21) Anmeldenummer: 05762875.2
(22) Anmeldetag: 04.08.2005
(51) Int. Cl.: A61M 25/06

(54) **INSERTIONSKOPF FÜR MEDIZINISCHE ODER PHARMAZEUTISCHE ANWENDUNGEN**
INSERTION HEAD FOR MEDICAL OR PHARMACEUTICAL APPLICATIONS
TETE D'INSERTION POUR APPLICATIONS MEDICALES OU PHARMACEUTIQUES

(30) Priorität: 13.08.2004 DE 102004039408
(43) Veröffentlichungstag der Anmeldung: 25.04.2007
(73) Patentinhaber: F. Hoffmann-La Roche AG, 4070 Basel (CH); Roche Diabetes Care GmbH, 68305 Mannheim (DE)
(72) Erfinder: SCHEURER, Simon, CH-3006 Bern (CH); HUNN, Marcel, CH-4900 Langenthal (CH); LINIGER, Jürg, CH-3072 Ostermundigen (CH)
(74) Vertreter: Rentsch Partner AG
(86) Internationale Anmeldenummer: PCT/CH2005/000459
(87) Internationale Veröffentlichungsnummer: WO 2006/015507

(56) Entgegenhaltungen:
- EP-A1- 1 256 355
- WO-A-02/081012
- WO-A2-02/098481
- WO-A2-03/011356
- US-A1- 2002 193 744
- US-A1- 2003 083 624
- US-A1- 2004 044 306
- US-B1- 6 261 259

## Beschreibung

Die Erfindung betrifft einen Insertionskopf für medizinische oder pharmazeutische Anwendungen, der auf einem organischen Gewebe, insbesondere der menschlichen Haut, platzierbar ist und eine Einstecheinrichtung aufweist, die in das Gewebe eindringt, wenn der Insertionskopf auf dem Gewebe platziert wird oder gegebenenfalls auch erst nachdem der Insertionskopf auf das Gewebe platziert wurde. Der Insertionskopf kann insbesondere Bestandteil eines Infusionssets für die Verabreichung eines Medikaments sein.

Aus der DE 198 21 723 C1 ist ein derartiger Insertionskopf bekannt. Der Insertionskopf umfasst ein Gehäuse, eine flexible Kanüle, die von einer Unterseite des Gehäuses abragt, und eine Einstechnadel, die dazu dient, die flexible Kanüle im Gewebe eines Patienten subkutan zu platzieren. Für das Einbringen in das Gewebe durchragt die Einstechnadel die Kanüle, und die Kanüle umschmiegt die Einstechnadel. Um einen Verwender des Insertionskopfs vor Stichverletzungen zu schützen, ist an dem Gehäuse lösbar ein Nadelschutz befestigt, der von dem Gehäuse die Kanüle und die Einstechnadel umgebend abragt. Die. von der Unterseite des Insertionskopf abragende Einstechnadel mit der umgebenden Kanüle und insbesondere auch der Nadelschutz vergrößern das Volumen des Insertionskopf und dadurch auch dessen Verpackung beträchtlich.

US 2002/0193744 A1 zeigt eine Nadelsicherheitsvorrichtung mit einem Kragen und einem daran schwenkbar verbundenen Gehäuse. Auf dem Kragen und den entsprechenden unteren Abschnitten des Gehäuses sind zusammenwirkende Verriegelungselemente vorgesehen. Wenn das Gehäuse in eine zum Kragen gefluchtete Position geschwenkt wird, verriegeln die Verriegelungselemente miteinander, so dass das Gehäuse am Kragen gehalten wird. Um sicherzustellen, dass die zusammenwirkenden Verriegelungselemente nicht dadurch sabotiert werden können, dass die Seitenwände des Gehäuses absichtlich vom Kragen weg gedrückt werden, sind am Kragen Barrieren in Form von Schutzklappen vorgesehen, welche die entsprechenden Abschnitte der Seitenwände abdecken.

Es ist eine Aufgabe der Erfindung, das Verpackungsvolumen für einen Insertionskopf, der eine Einstecheinrichtung und eine Schutzeinrichtung für die Einstecheinrichtung aufweist, zu verringern.

Die Erfindung geht von einem Insertionskopf für medizinische oder pharmazeutische Angemäß Anspruch 1 aus, der ein Gehäuse mit einer auf organischem Gewebe platzierbaren Unterseite und eine Einstecheinrichtung aufweist. Die Einstecheinrichtung umfasst einen Einstechabschnitt mit einer Spitze zum Eindringen in das Gewebe. Der Einstechabschnitt ragt vorzugsweise von der Unterseite des Gehäuses vor. Grundsätzlich kann er jedoch stattdessen auch von einer Seite des Gehäuses vorragen, solange er für ein Eindringen in das Gewebe über die Unterseite ausreichend weit vorragt. Der Einstechabschnitt ragt vorzugsweise mit einer an subkutane Applikationen angepassten Länge über die Unterseite des Gehäuses vor, vorzugsweise unmittelbar von der Unterseite ab. Für Applikationen innerhalb der Haut oder in intramuskulärem Gewebe ist der Einstechabschnitt entsprechend kürzer oder länger. Als Einstechabschnitt wird derjenige Längenabschnitt der Einstecheinrichtung verstanden, der in der Applikation in das Gewebe ragt.

Der Insertionskopf ist vorzugsweise Bestandteil eines Infusionssets für die Verabreichung von Insulin, eines Schmerzmittels oder eines anderen per Infusion verabreichbaren Medikaments. Anstatt für eine Medikamentenverabreichung oder grundsätzlich auch eines anderen verabreichbaren Produkts kann der Insertionskopf auch zu Diagnosezwecken dienen. In solchen Applikationen kann der Einstechabschnitt Träger eines Sensors zum Messen von beispielsweise der Glukosekonzentration in einer Körperflüssigkeit oder einer anderen physikalischen und/oder biochemischen Größe dienen, die für den Gesundheitszustand eines Patienten maßgeblich ist oder sein kann. Der Insertionskopf kann zu Diagnosezwecken auch als Perfusionsvorrichtung gebildet sein. In solch einer Ausbildung wird der Einstechabschnitt nach dem Einbringen in das Gewebe von einer Spülflüssigkeit durchströmt, die ein oder mehrere bestimmte Inhaltsstoffe der Körperflüssigkeit bei dem Durchströmen aufnimmt, um die mit dem betreffenden Inhaltsstoff oder die mehreren Inhaltsstoffen angereicherte Spülflüssigkeit zu analysieren. Schließlich kann der Insertionskopf eine Vorrichtung für die Verabreichung eines Produkts und eine Diagnoseeinrichtung in Kombination bilden. Der Einstechabschnitt kann für die Zuführung eines Produkts, das insbesondere ein Medikament oder eine Spülflüssigkeit sein kann, oder die Abführung einer Körperflüssigkeit oder nur eines oder mehrerer bestimmter Inhaltsstoffe einer Körperflüssigkeit gebildet sein, d.h. der Einstechabschnitt bildet in solch einer Applikation wenigstens einen Strömungsquerschnitt. Der Einstechabschnitt kann der Zu- und Abführung von Stoffen auch in Kombination dienen. Ist der Insertionskopf nur als Messvorrichtung gebildet, so kann der Einstechabschnitt auch nur dazu dienen, einen Sensor oder einen Teil eines Sensors zu platzieren, d.h. rein als mechanische Einbringeinrichtung. In einer Weiterbildung als Messvorrichtung kann er über das mechanische Einbringen hinaus auch der Übertragung von Steuersignalen zu dem Sensor und/oder von Messsignalen von dem Sensor dienen. In kombinierten Applikationen kann er schließlich über wenigstens einen Strömungsquerschnitt für den Stofftransport, d.h. eine Strömungsleitung, und wenigstens eine Signalleitung verfügen. Auf die Signalleitung kann verzichtet werden, wenn der Sensor für den drahtlosen Empfang von Steuersignalen und/oder das drahtlose Senden von Messsignalen eingerichtet ist. Schließlich kann die Einstechrichtung auch zwei oder mehr Einstechabschnitte aufweisen, die separat abragen. So kann ein erster Einstechabschnitt dem Stofftransport in das Gewebe und ein anderer, zweiter Einstechabschnitt dem Stofftransport aus dem Gewebe oder nur dem Einbringen eines Sensors oder eines Teils eines Sensors dienen. Mit mehreren Einstechabschnitten, die je einen Strömungsabschnitt aufweisen, können mit dem gleichen Insertionskopf auch unterschiedliche Stoffe verabreicht werden. Dies kann auch mit einem Einstechabschnitt verwirklicht werden, der mehrere, separate Strömungsquerschnitte bildet.

Um den Verwender vor Stichverletzungen zu schützen, ist eine Schutzeinrichtung vorgesehen, die zumindest die Spitze des Einstechabschnitts, vorzugsweise den gesamten Einstechabschnitt, in einem ersten Zustand des Insertionskopfs verdeckt. In einem zweiten Zustand, in den der Insertionskopf für die Applikation bringbar ist, liegt die Spitze des Einstechabschnitts frei und kann in das Gewebe eingestochen werden.

Nach der Erfindung bildet das Gehäuse selbst die Schutzeinrichtung, d.h. der Insertionskopf ist mit einer integrierten Schutzeinrichtung ausgestattet. Die Einstecheinrichtung kann relativ zu dem Gehäuse wenigstens zwei vorgegebene Positionen einnehmen, nämlich eine erste Position im ersten Zustand des Insertionskopfs und eine zweite Position im zweiten Zustand des Insertionskopfs. Die Einstecheinrichtung wird in beiden Positionen von dem Gehäuse gehalten, d.h. sie ist in beiden Positionen mit dem Gehäuse ausreichend fest verbunden, um mit dem Gehäuse hinsichtlich der Handhabung eine Einheit zu bilden. Die erste Position der Einstecheinrichtung wird im Folgenden als Schutzposition und die zweite Position wird als Einstechposition bezeichnet.

Die Einstecheinrichtung ist mit dem Gehäuse so verbunden, dass der Einstechabschnitt relativ zu dem Gehäuse schwenkbar ist. Dabei kann der Einstechabschnitt in das Gehäuse eingeschwenkt werden, so dass zumindest seine Spitze, vorzugsweise der gesamte Einstechabschnitt, hinter dem Gehäuse, vorzugsweise dessen Unterseite, zurücksteht und dadurch vom Gehäuse verdeckt wird.

In der Schutzposition nimmt der Einstechabschnitt eine im Vergleich zu der Einstechposition gekippte Lage ein, so dass das Gehäuse die Spitze des Einstechabschnitts verdeckt.

In bevorzugten Ausführungen ist die Einstecheinrichtung mit dem Gehäuse bewegbar permanent verbunden. Dass die Einstecheinrichtung mit dem Gehäuse permanent verbunden ist, bedeutet, dass sie zumindest für die Bewegung aus der Schutzposition in die Einstechposition und/oder aus der Einstechposition in die Schutzposition nicht von dem Gehäuse gelöst wird. Vorzugsweise ist die Einstecheinrichtung sogar in dem Sinne permanent mit dem Gehäuse verbunden, dass sie von dem Gehäuse gar nicht gelöst werden kann, zumindest nicht ohne eine besondere Kraftanstrengung oder nur durch Zerstörung.

In der erfindungsgemässen Ausführung, in der der Einstechabschnitt relativ zu dem Gehäuse schwenkbar ist, bilden die Einstecheinrichtung und das Gehäuse miteinander ein Gelenk mit einer quer zu dem Einstechabschnitt weisenden Gelenkachse. Die Einstecheinrichtung ist um die Gelenkachse relativ zu dem Gehäuse drehbar. Für die Ausbildung eines derartigen Gelenks kann das Gehäuse oder die Einstecheinrichtung eine Lagerbuchse und die andere Komponente den Lagerzapfen bilden. Die Lagerbuchse muss den Lagerzapfen über einen Winkel von mehr als 180° umgeben, wenn die Einstecheinrichtung von dem Gehäuse fest gehalten werden soll.

Obgleich es grundsätzlich genügt, wenn die Einstecheinrichtung nur in eine Richtung, d.h. einmalig, relativ zu dem Gehäuse bewegbar ist, dann vorzugsweise aus der Schutzposition in die Einstechposition, wird es bevorzugt, wenn die Einstecheinrichtung zwischen den beiden Positionen wahlfrei hin und her bewegbar ist, so dass die Einstecheinrichtung für einen Gebrauch in die Einstechposition und für die Entsorgung wieder in die Schutzposition bewegt werden kann. Die Verbindung zwischen der Einstecheinrichtung und dem Gehäuse ist oder umfasst vorzugsweise einen Formschluss, der insbesondere ein Gelenk bilden kann. Die Verbindung kann alternativ jedoch auch rein kraftschlüssig sein oder sogar stoffschlüssig, beispielsweise dann, wenn der Einstechabschnitt in die Schutzposition gebogen wird.

Die Einstecheinrichtung wird in ihren wenigstens zwei unterschiedlichen Positionen ausreichend fest gehalten. Dies bedeutet, dass der Verwender beim Einstechen nicht den Einstechabschnitt halten muss, sondern lediglich das Gehäuse oder eine das Gehäuse beim Einstechen führende Insertionshilfseinrichtung, so dass der Einstechabschnitt bei der Platzierung des Gehäuses auf dem Gewebe automatisch in das Gewebe eindringt. Auch in der Schutzposition wird die Einstecheinrichtung gehalten, so dass sie nicht bereits bei leichten Erschütterungen die Schutzposition verlässt. In bevorzugten Ausführungen verrastet die Einstecheinrichtung in jeder der vorgegebenen Positionen mit dem Gehäuse. Die Rastverbindung in der Schutzposition ist lösbar. Die Rastverbindung in der Einstechposition kann lösbar oder unlösbar sein. Die Rastverbindung kann von einem Verbindungsgelenk selbst gebildet werden, beispielsweise indem der genannte Tragzapfen und dessen Lagerbuchse aneinander angepasste polygonale Querschnitte aufweisen. Rastelemente können stattdessen oder zusätzlich jedoch auch außerhalb der jeweiligen Gelenkverbindung vorgesehen sein.

In einer bevorzugten Weiterbildung ist die Einstecheinrichtung mit dem Gehäuse so verbunden, dass sie zwei oder mehr vorgegebene Einstechpositionen einnehmen kann, wobei jede der Einstechpositionen vorzugsweise als Rastposition vorgegeben wird. So kann der Einstechabschnitt in einer der Einstechpositionen über die Unterseite des Gehäuses senkrecht, d.h. in einem rechten Winkel vorragen und in einer anderen Einstechposition mit der Unterseite einen Winkel kleiner als 90° einschließen, vorzugsweise einen Winkel kleiner als 45°. Der Verwender kann zwischen den vorgegebenen Einstechpositiönen frei wählen. Falls die unterschiedlichen Positionen der Einstecheinrichtung oder nur die unterschiedlichen Einstechpositionen rein kraftschlüssig vorgegeben werden, so dass die Einstecheinrichtung in den betreffenden Positionen rein kraftschlüssig am Gehäuse gehalten wird, kann der Einstechabschnitt relativ zu der Unterseite des Gehäuses in jede beliebige Winkelposition zwischen 0 und 90° gebracht werden.

Für die Verbindung mit dem Gehäuse umfasst die Einstecheinrichtung vorzugsweise eine Verbindungsstruktur, von der der Einstechabschnitt vorragt. Die Verbindungsstruktur bildet in der Einstechposition in bevorzugten Ausführungen einen Teil der Unterseite des Insertionskopfs, gegebenenfalls auch die gesamte Unterseite, und liegt mit ihrer Unterseite oder einem Teil ihrer Unterseite, von dem der Einstechabschnitt abragt, auf dem Gewebe auf, wenn der Einstechabschnitt vollständig in das Gewebe eingeführt ist. Grundsätzlich kann die Unterseite der Verbindungsstruktur jedoch auch ein kleines Stück weit hinter der Kontaktfläche des Insertionskopfs zurückstehen.

Das Gehäuse bildet die integrierte Schutzeinrichtung vorzugsweise in Form einer Aufnahme, die zu einer Außenseite des Gehäuses hin offen ist, so dass der Einstechabschnitt in dieser Aufnahme geschwenkt oder zumindest mit seiner Spitze in die Aufnahme gebogen werden kann. Denkbar ist es grundsätzlich auch, dass die Einstecheinrichtung in dem ersten Zustand des Insertionskopfs in der Aufnahme oder zumindest mit ihrer Spitze in der Aufnahme aufgenommen und in der Schutzposition lösbar am Gehäuse befestigt ist, vorzugsweise mittels Rastverbindung, und von dem Gehäuse lösbar und in der Einstechposition wieder mit dem Gehäuse verbindbar ist, vorzugsweise mittels Rastverbindung. Die Aufnahme ist vorzugsweise an der Unterseite des Gehäuses gebildet. Vorteilhaft ist es ferner, besonders für einen einschwenkenden Einstechabschnitt, wenn die Aufnahme nicht nur zu der betreffenden Außenseite hin offen ist, vorzugsweise der Unterseite, sondern auch zu einer Gelenkverbindung der Einstecheinrichtung. Die Aufnahme kann auch zur Unterseite und zu einer anderen Außenseite hin offen sein, um beispielsweise Schwenkbewegungen um unterschiedliche Schwenkachsen zu ermöglichen.

Der Einstechabschnitt kann insbesondere ein flexibles Element, beispielsweise in Form einer flexiblen Kanüle, und ein Stabilisierungselement umfassen oder allein aus diesen Teilen gebildet sein. Das Stabilisierungselement dient in derartigen Ausführungen zur Stabilisierung des flexiblen Elements während des Einstechens und kann nach dessen Einbringung in das Gewebe vorteilhafterweise entfernt werden, um im Gewebe während der Verwendung möglichst wenig Irritationen hervorzurufen. Das Stabilisierungselement bildet vorzugsweise eine Einstechkante des Einstechabschnitts.

Den Einstechabschnitt kann auch eine Einstechnadel, insbesondere Stahlnadel, bilden, die während der Applikation im Gewebe verbleibt. In bevorzugt subkutanen Applikationen sollte insbesondere ein derartiger Einstechabschnitt möglichst kurz sein, vorzugsweise höchstens 12 mm, noch bevorzugter höchstens 8 mm. Die Nadel sollte einerseits für den Verbleib im Gewebe möglichst flexibel, d. h. elastisch biegbar, muss aber andererseits doch so steif sein, dass sie in und vorzugsweise durch die menschliche Haut dringen kann.

In anderen bevorzugten Ausführungen verringert sich die Steifigkeit des Einstechabschnitts automatisch, wenn der Einstechabschnitt in das Gewebe eingebracht ist. So kann der Einstechabschnitt beispielsweise aus einem Material gefertigt sein, das im Bereich der Körpertemperatur, beispielsweise bei Überschreiten einer Temperatur von 35 oder 36°C, vorzugsweise noch darunter, nämlich im Bereich von 32° bis 34° C, deutlich biegsamer als bei tieferen Temperaturen ist. So kann der Einstechabschnitt insbesondere so wie in PCT/EP 04/00310, der PCT/EP 04/00309, der DE 101 17 286 A oder der DE 10 2004 002 472 beschrieben gebildet sein. Besonders vorteilhaft ist ein Einstechabschnitt, der gänzlich oder zumindest teilweise aus einem Polymermaterial gebildet ist, das bei Kontakt mit Körperflüssigkeit weicher und der Einstechabschnitt dadurch biegsamer wird, wobei das Weicherwerden vorzugsweise allein auf einer Absorption von Wasser und einer damit verbundenen Lockerung von Sekundärbindungen des Polymermaterials beruht. Grundsätzlich kann das Polymermaterial jedoch zum Teil auch gelöst und dadurch die Biegsamkeit erhöht werden. Vorteilhaft ist auch die Verwendung eines durch Absorption erweichbaren Polymermaterials, das im genannten Temperaturbereich aufgrund des Effekts der Temperatur zusätzlich erweicht. In derartigen Ausbildungen kann auf ein zusätzliches Stabilisierungselement verzichtet werden, und der Einstechabschnitt ist im Gewebe dennoch flexibler als eine herkömmliche Einstechnadel.

Der Insertionskopf kann Bestandteil eines Infusions- und/oder Diagnosesets sein, das über den Insertionskopf hinaus auch eine Zu- und/oder Ableitung für einen Stofftransport und/oder eine Signalübermittlung umfasst. Das Gehäuse bildet für solch eine Zu- und/oder Ableitung vorteilhafterweise einen Stützabschnitt, der insbesondere als Auflage gebildet sein kann. Falls die Zu- und/oder Ableitung wie bevorzugt lösbar mit dem Gehäuse oder der Einstecheinrichtung verbunden ist oder wird, stützt der Stützabschnitt des Gehäuses vorteilhafterweise einen Konnektor, an den die Zu- und/oder Ableitung angeschlossen ist. Vorteilhaft ist es, wenn der Stützabschnitt eine im platzierten Zustand des Insertionskopfs parallel zu der Gewebeoberfläche weisende Führung für den Konnektor bildet, so dass der Konnektor in Richtung der Gewebeoberfläche entlang der Führung gleitend fluidisch und/oder signaltechnisch mit dem Gehäuse oder vorzugsweise unmittelbar der Einstecheinrichtung verbunden werden kann. Mechanisch wird der Konnektor vorzugsweise mit dem Gehäuse verbunden.

Falls die Einstecheinrichtung zusätzlich zu einem flexiblen Element ein Stabilisierungselement, beispielsweise eine Einstechnadel, umfasst, ist vorzugsweise ein Griffteil des Stabilisierungselements in dem Stützabschnitt aufgenommen, wenn die Einstecheinrichtung ihre Schutzposition einnimmt. Das Griffteil des Stabilisierungselements und der Konnektor nutzen in derartigen Ausführungen den gleichen Stützabschnitt des Gehäuses.

Bevorzugte Merkmale, die die vorstehend beschriebenen Ausgestaltungen ergänzen oder durch diese ergänzt werden können, werden auch in den Unteransprüchen und deren Kombinationen beschrieben.

Nachfolgend wird anhand von Figuren ein bevorzugtes Ausführungsbeispiel der Erfindung beschrieben. An dem Ausführungsbeispiel offenbar werdende Merkmale können je einzeln und in jeder Kombination die Gegenstände der Ansprüche und auch die vorstehend beschriebenen Ausgestaltungen vorteilhaft weiterbilden. Es zeigen:
- Figur 1: einen Insertionskopf eines ersten Ausführungsbeispiels mit einer Einstecheinrichtung in einer Einstechposition vor dem Einbringen in organisches Gewebe,
- Figur 2: den Insertionskopf mit der Einstecheinrichtung in einer Schutzposition,
- Figur 3: den Insertionskopf mit der in einer Ansicht dargestellten Einstecheinrichtung in der Schutzposition,
- Figur 4: den Insertionskopf mit der Einstecheinrichtung in der Einstechposition und einer konnektierten Fluidzuleitung,
- Figur 5: einen Insertionskopf eines zweiten Ausführungsbeispiels in einem Längsschnitt und
- Figur 6: den Insertionskopf des zweiten Ausführungsbeispiels in einer Ansicht.

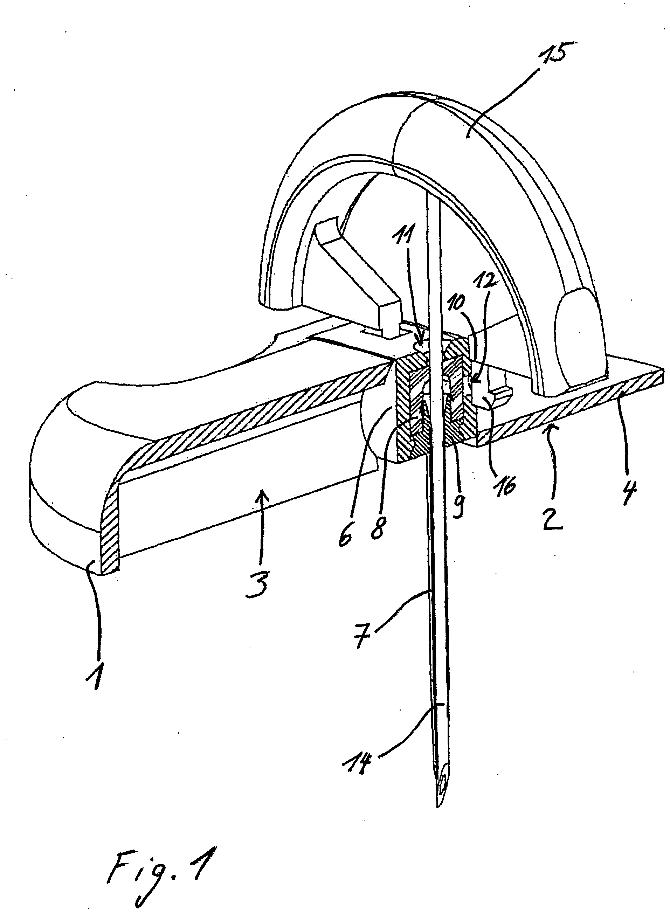

Figur 1 zeigt in einem ersten Ausführungsbeispiel einen Insertionskopf mit einem Gehäuse 1 in einem Längsschnitt und einer Einstecheinrichtung, die teilweise in der gleichen Schnittebene dargestellt ist und relativ zu dem Gehäuse 1 eine Einstechposition einnimmt. Die Einstecheinrichtung umfasst eine Verbindungsstruktur 6, die mit dem Gehäuse 1 bewegbar permanent verbunden ist. Die Einstecheinrichtung umfasst ferner ein flexibles Eindringelement 7, das im Ausführungsbeispiel als Kanüle mit einem inneren Strömungsquerschnitt gebildet ist, den in der Verwendung des Insertionskopfs 1 als Infusionskopf eines Infusionssets ein fluides Infusat, beispielsweise Insulin, durchströmt. Das Eindringelement 7 ist so flexibel, dass es ohne eine zusätzliche Stabilisierung die Haut eines Patienten nicht durchdringen und auch erst gar nicht in die Haut eindringen kann. Für das Einbringen des Eindringelements 7 durchragt ein ausreichend biegesteifes Stabilisierungselement 14 in Form einer Einstechnadel die Verbindungsstruktur 6 und das davon abragende Eindringelement 7, so dass das Stabilisierungselement 14 mit einer freien Spitze über das freie Ende des flexiblen Eindringelements 7 vorsteht. Das flexible Eindringelement 7 schmiegt sich mit einer leichten Spannung an die Mantelaußenfläche des Stabilisierungselements 14 an.

Das Eindringelement 7 und das Stabilisierungselement 14 bilden einen Einstechabschnitt der Einstecheinrichtung, der in der in Figur 1 gezeigten Einstechposition über eine Unterseite 2 des Gehäuses 1 rechtwinklig vorragt. Für die Applikation kann der Insertionskopf mit seiner Unterseite 2 in Richtung auf die Haut bewegt werden, wobei das Stabilisierungselement 14 in die Haut einsticht und der Einstechabschnitt 7, 14 in und durch die Haut eindringt bis der Insertionskopf mit seiner Unterseite 2 die Hautoberfläche kontaktiert. Die Unterseite 2 ist vorzugsweise als Adhäsionsfläche gebildet, die mit einer Abziehfolie bedeckt ist, die für die Applikation abgezogen wird, um die Adhäsionsfläche freizulegen. Sobald die Unterseite 2 die Hautoberfläche kontaktiert, wird daher automatisch eine Adhäsionsverbindung hergestellt.

Das Stabilisierungselement 14 ist mit einem Griffteil 15 versehen. Das Griffteil 15 hat die Form eines Halbrings. Das Stabilisierungselement 14 ragt von einem zentralen Bereich von der Innenseite des Griffteils 15 ab und über die Ringenden des Griffteils 15 hinaus. In den beiden äußeren Endbereichen des Griffteils 15 ragt von dessen Innenseite nach radial einwärts je ein Verbindungselement 16 vor, das der Befestigung des Griffteils 15 und damit gemeinsam des Stabilisierungselements 14 an der Verbindungsstruktur 6 dient. Die beiden Verbindungselemente 16 des Griffteils 15 bilden je ein hakenförmiges Rastelement, das mit einem entsprechenden Gegenelement in einer Ausnehmung der Verbindungsstruktur 6 lösbar verrastet ist. Der Rasteingriff der beiden Verbindungselemente 16 kann gelöst werden, indem die beiden Ringenden des Griffteils 15 gegen dessen rückstellende Elastizität auseinander zu gedrückt werden und das Griffteil 15 dann von der Verbindungsstruktur 6 weg bewegt wird. Dabei wird das Stabilisierungselement 14 aus dem flexiblen Eindringelement 7 herausgezogen, so dass nach dem Entfernen des Stabilisierungselements 14 nur noch das Eindringelement 7 im Gewebe verbleibt.

Die Verbindungsstruktur 6 bildet einen Hohlraum, der an einer Seite, von der das Eindringelement 7 abragt, offen ist und ferner der offenen Seite gegenüber einen Durchgang 11 für das Stabilisierungselement 14 und ferner einen zweiten Durchgang 12 für den Anschluss einer Infusatzuleitung aufweist. In dem Hohlraum ist ein Dichtelement 8 angeordnet, das die beiden Durchgänge 11 und 12 abdichtet. Das flexible Eindringelement 7 ragt von einem Halter 9 ab, der den Hohlraum der Verbindungsstruktur 6 von dem Strömungsquerschnitt des Eindringelements 7 abgesehen an seiner offenen Seite verschließt und dort form- und kraftschlüssig an der Verbindungsstruktur 6 gehalten ist. Gleichzeitig hält der Halter 9 auch das Dichtelement 8 innerhalb des Hohlraums der Verbindungsstruktur 6 in Position, so dass es gegen die beiden Durchgänge 11 und 12 drückend diese dicht verschließt. Das Dichtelement 8 bildet zusammen mit dem Halter 9 im Hohlraum eine Kammer 10, durch die bei einer Infusion das Infusat in das flexible Eindringelement 7 strömt. Das Dichtelement 8 ist so gebildet, dass es von dem Stabilisierungselement 14 durchstochen werden kann und sowohl im durchstochenen Zustand als auch nach dem Herausziehen des Stabilisierungselements 14 die Durchgänge 11 und 12 abdichtet.

Das Gehäuse 1 weist in der gezeigten Schnittebene drei Abschnitte auf. In einem mittleren der drei Abschnitte hält das Gehäuse 1 die Verbindungsstruktur 6. Zu der einen Seite des Mittelabschnitts bildet das Gehäuse 1 eine Aufnahme 3 in Form eines Hohlraums, der sich zu der Unterseite 2 des Gehäuses 1 und zu der Verbindungsstruktur 6 hin öffnet. Die Verbindungsstruktur 6 wird von dem Gehäuse 1 so gehalten, dass sie hinter der Unterseite 2 ein kleines Stück weit zurücksteht. Zu der anderen Seite des Mittelabschnitts bildet das Gehäuse 1 einen Stützabschnitt 4 in Form eines platten- oder scheibenförmigen Flachteils.

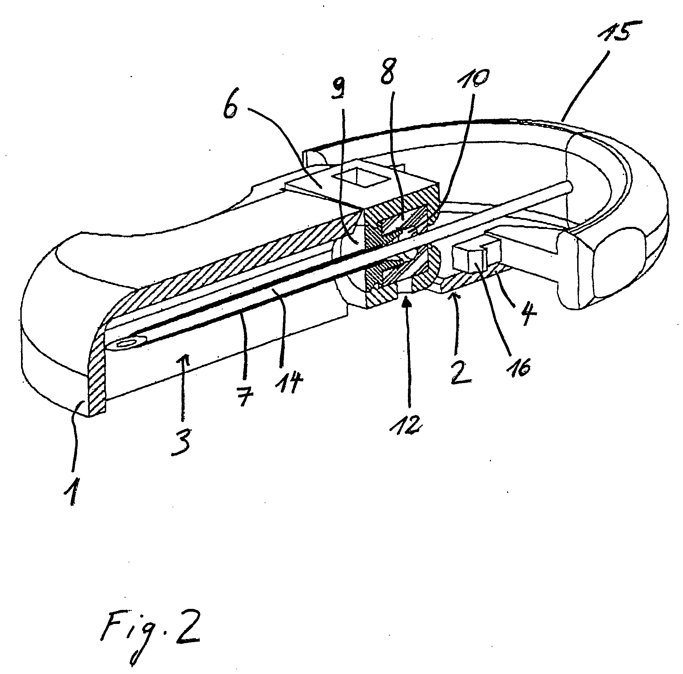

Figur 2 zeigt den Insertionskopf in der gleichen Schnittdarstellung wie Figur 1, wobei die Einstecheinrichtung allerdings eine Schutzposition einnimmt, in der ihr gesamter Einstechabschnitt 7, 14 in der Aufnahme 3 aufgenommen ist. Die Überführung aus der Einstechposition in die Schutzposition erfolgt durch eine Drehbewegung der Verbindungsstruktur 6 um eine quer zu dem Einstechabschnitt 7, 14 weisende Drehachse.

Durch die Drehbewegung der Verbindungsstruktur 6 wird der Einstechabschnitt 7, 14 in den Hohlraum 3 geschwenkt. Das über die Verbindungsstruktur 6 hinaus dem Einstechabschnitt 7, 14 gegenüberliegende Griffteil 15 dient dem Verwender nicht nur als Griff zum Herausziehen des Stabilisierungselements 14, sondern auch als Schwenkgriff. In der Schutzposition der Einstecheinrichtung liegt das Griffteil 15 auf der Oberseite des Stützabschnitts 4 auf, der somit für das Griffteil 15 eine Auflage bildet.

Mit der in ihrer Schutzposition befindlichen Einstecheinrichtung wird der Insertionskopf ausgeliefert. Figur 2 zeigt den Insertionskopf somit im Auslieferungszustand. Der Insertionskopf weist in diesem Zustand nur eine geringe, auf die Unterseite 2 gemessene Höhe auf. Entsprechend klein kann das Volumen der sterilen Verpackung des Insertionskopfs sein. Die in der Fläche der Unterseite 2 gemessene Länge und Breite des Insertionskopfs kann den entsprechenden Abmessungen herkömmlicher Insertionsköpfe entsprechen. Die Mindesthöhe wird von der Verbindungsstruktur 6 vorgegeben, die mit den Anschlüssen 11 und 12 für das Stabilisierungselement 14 und eine Infusatzuleitung, mit einer Verbindungseinrichtung für die Verbindung mit dem Gehäuse 1 und mit einer Verbindungseinrichtung für das Griffteil 15 versehen ist. Der Stützabschnitt 4 und das darauf geschwenkte Griffteil 15 weisen zusammen in etwa die Höhe des mit der Aufnahme 3 versehenen Gehäuseabschnitts auf.

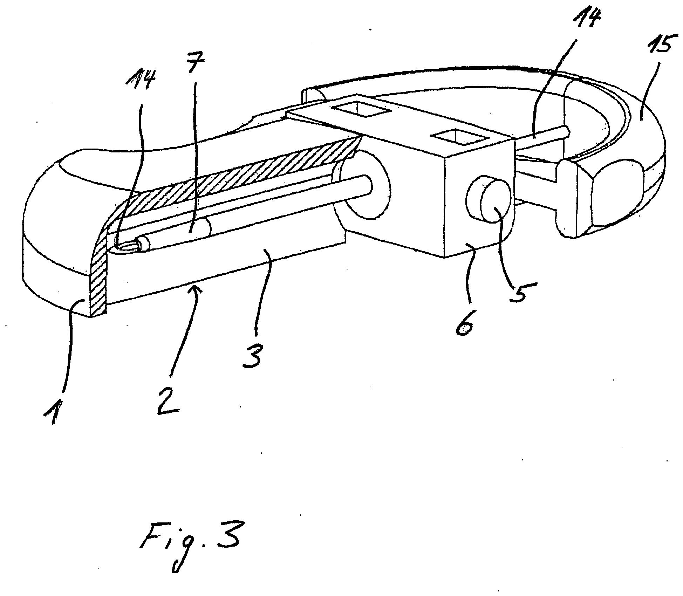

Figur 3 zeigt den Insertionskopf mit der in ihrer Schutzposition befindlichen Einstecheinrichtung. Während der den Hohlraum 3 bildende Gehäuseabschnitt wieder im Längsschnitt der Figuren 1 und 2 dargestellt ist, zeigt Figur 3 die Verbindungsstruktur 6 komplett in einer perspektivischen Ansicht. Die Verbindungsstruktur 6 bildet an zwei einander gegenüberliegenden Seiten je einen Gelenkzapfen 5. Die beiden Gelenkzapfen 5 ragen quer zu dem Einstechabschnitt 7, 14 ab. Das Gehäuse 1 bildet für jeden der Gelenkzapfen 5 je eine Gelenkbuchse. Die Verbindungsstruktur 6 und das Gehäuse 1 bilden somit je ein Gelenkelement eines einfachen Drehgelenks mit einer einzigen Gelenkachse, um die die Verbindungsstruktur 6 verdrehbar und der Einstechabschnitt 7, 14 dadurch verschwenkbar mit dem Gehäuse 1 verbunden ist. Die Verbindungsstruktur 6 kann daher auch als Drehkopf bezeichnet werden. Die Verbindung mit dem Gehäuse 1 ist ferner so gestaltet, dass die Verbindungsstruktur 6 sowohl in der Schutzposition als auch in der Einstechposition je eine Rastverbindung mit dem Gehäuse 1 bildet.

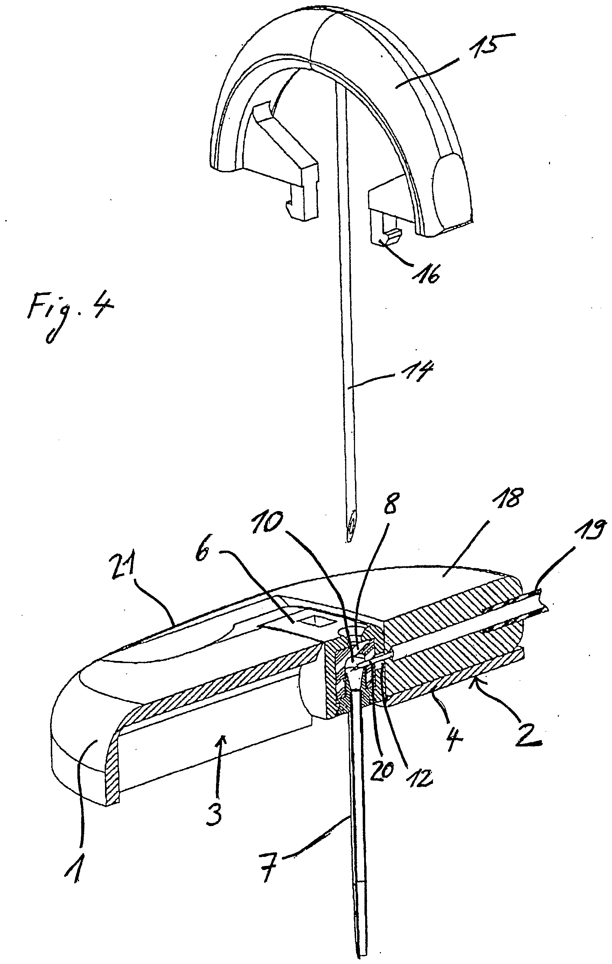

Figur 4 zeigt den Insertionskopf mit angeschlossener Infusatzuleitung 19. Die Zuleitung 19 kann insbesondere von einem flexiblen Katheter gebildet werden. Das Stabilisierungselement 14 ist von der Verbindungsstruktur 6 gelöst und kann beispielsweise in der Verpackung des Insertionskopfs abgelegt werden.

Die Infusatzuleitung 19 ist fest mit einem Konnektor 18 verbunden, der dem Anschluss der Infusatzuleitung 19 an die Verbindungsstruktur 6 und dadurch an das flexible Eindringelement 7 dient. Der Konnektor 18 bildet zwei Verbindungselemente 21 für die mechanische Befestigung am Gehäuse 1, von denen im Längsschnitt der Figur 4 eines gezeigt ist. Alternative könnte die Formschlussverbindung des Konnektors 18, entsprechende Modifizierung vorausgesetzt, mit der Verbindungsstruktur 6 gebildet sein. Die in Figur 4 gezeigte Längsschnittebene bildet eine Symmetrieebene des Insertionskopfs einschließlich des Konnektors 18. Die beiden Verbindungselemente21 sind biegeelastisch und an ihren Enden je mit einem Rastelement in Form eines Hakens versehen. Der Konnektor 18 weist ferner eine der Oberseite des Stützabschnitts 4 entsprechend geformte Unterseite auf, so dass der Konnektor über der Oberseite des Stützabschnitts 4 gleitend auf die Verbindungsstruktur 6 zu geschoben werden kann, bis der Anschluss an die Einstecheinrichtung und eine Rastverbindung mit dem Gehäuse 1 hergestellt sind. Für den fluidischen Anschluss bildet der Konnektor 18 eine Verbindungsnadel 20, die zumindest im Wesentlichen parallel zur Unterseite des Konnektors 18 von diesem in dessen Vorschubrichtung vorsteht und bei dem Vorschieben des Konnektors 18 bis gegen die Verbindungsstruktur 6 deren Dichtelement 8 durchdringt, so dass die Verbindungsnadel 20 mit ihrer Spitze in die Kammer 10 ragt. Die Verbindungsnadel 20 ist hohl, und der Konnektor 18 bildet einen Leitungsquerschnitt, der die Infusatzuleitung 19 mit der Verbindungsnadel 20 verbindet. Auf diese Weise ist nach dem Anschluss eine fluiddichte Verbindung von der Infusatzuleitung 19 bis zu dem freien Ende des Eindringelements 7 geschaffen.

Der typische Applikationsverlauf des Insertionskopfs gestaltet sich wie folgt:
Der Insertionskopf gelangt in dem in Figur 2 gezeigten Auslieferungszustand steril verpackt zum Verwender. Der Verwender öffnet die Verpackung und entnimmt den Insertionskopf. Aufgrund der vom Gehäuse 1 in Form der Aufnahme 3 gebildeten integrierten Schutzeinrichtung und der in ihrer Schutzposition befindlichen Einstecheinrichtung sind der Verwender vor Stichverletzungen und der Einstechabschnitt 7, 14 vor Beschädigung geschützt.

In einem nächsten Schritt wird die Abziehfolie von der Unterseite 2 des Gehäuses 1 abgezogen, so dass die Adhäsionsfläche der Unterseite 2 frei liegt. Für die Applikation hält der Verwender mit der einen Hand das Gehäuse 1 und greift mit der anderen Hand das Griffteil 15. Er schwenkt das Griffteil 15 um die vom Drehgelenk gebildete Drehachse und dadurch die Einstecheinrichtung in die in Figur 1 gezeigte Einstechposition, in der der Einstechabschnitt 7, 14 über die Unterseite 2 des Gehäuses vorragt. In diesem Zustand drückt der Verwender den Insertionskopf gegen die Haut, so dass der Einstechabschnitt 7, 14 in die Haut eindringt und die Haut durchdringt, bis der Insertionskopf mit seiner Unterseite 2 die Hautoberfläche kontaktiert und dadurch an der Hautoberfläche durch Adhäsionskräfte haftet. Das freie Ende des flexiblen Eindringelements 7 wird durch das Einstechen automatisch im Gewebe platziert, vorzugsweise subkutan. Das Stabilisierungselement 14 kann nun entfernt werden, indem der Verwender die beiden Ringenden des Griffteils 15 zusammendrückt, dessen Verbindungselemente 16 dadurch aus dem Eingriff mit der Verbindungsstruktur 6 bringt und das Griffteil 15 mit dem daran befestigen Stabilisierungselement 14 aus der Verbindungsstruktur 6 und insbesondere aus dem Eindringelement 7 herauszieht. Das Dichtelement 8 sorgt dafür, dass der Strömungsquerschnitt des Eindringelements 7 auch nach dem Herausziehen von der Umgebung steril abgedichtet ist. Der Insertionskopf ist nun bereit für den Anschluss der Infusatzuleitung 19 (Figur 4). Für den Anschluss wird der Konnektor 18 auf dem Stützabschnitt 4 des Gehäuses 1 aufliegend mit der Verbindungsnadel 20 voran auf die Verbindungsstruktur 6 vorgeschoben. Im Zuge dieser Schiebebewegung dringt die Verbindungsnadel 20 durch den Durchgang 12 und das Dichtelement 8 in die Kammer 10 ein. Gleichzeitig gelangen die Verbindungselemente 21 des Konnektors 18 in Rasteingriff mit dem Gehäuse 1, so dass zum einen der fluidische Anschluss und zum anderen die mechanische Verbindung der Infusatzuleitung 19 an das und mit dem flexiblen Eindringelement 7 hergestellt ist, wie in Figur 4 gezeigt.

Falls die Abziehfolie im Bereich der Aufnahme 3 eine Aussparung aufweist, kann die Abziehfolie auch erst nach dem Ausschwenken des Einstechabschnitts 7, 14 abgezogen werden.

Nach der Applikation wird der Konnektor 18 von dem Gehäuse 1 gelöst. Der Insertionskopf wird von der Hautoberfläche gelöst und dabei das flexible Eindringelement 7 aus dem Gewebe gezogen.

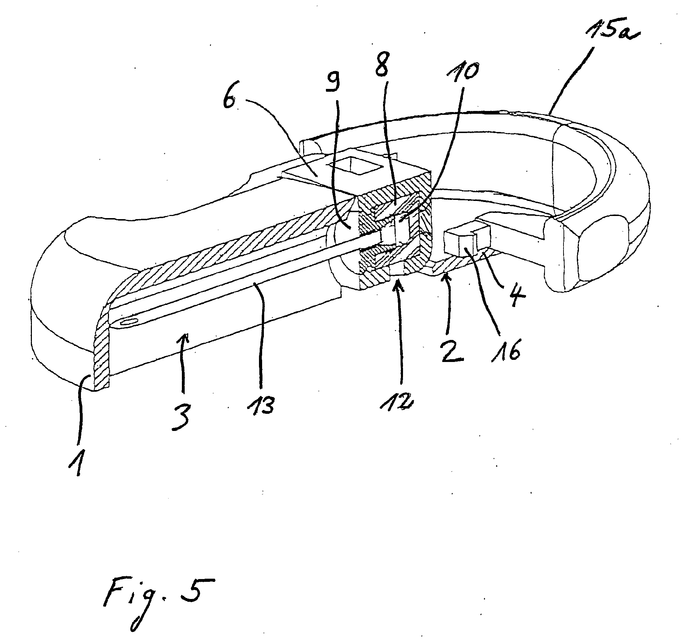

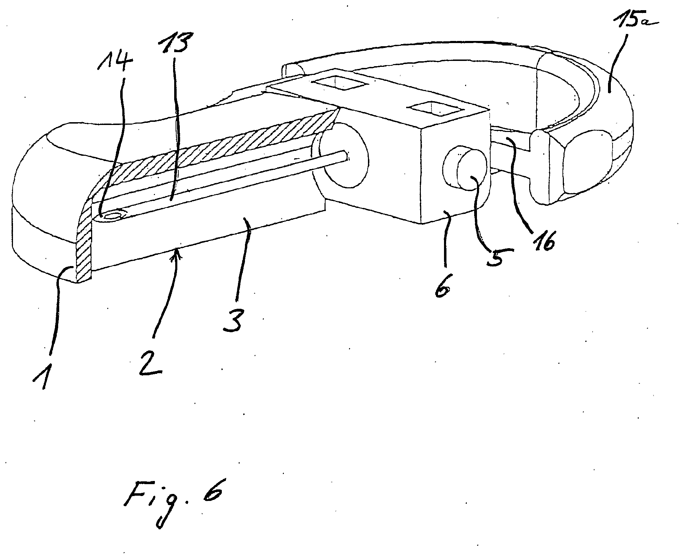

Figur 5 zeigt einen Insertionskopf eines zweiten Ausführungsbeispiels mit einer in der Schutzposition befindlichen Einstecheinrichtung in einem Längsschnitt. Figur 6 zeigt den Insertionskopf des zweiten Ausführungsbeispiels im gleichen Zustand in einer Ansicht.

Der Insertionskopf des zweiten Ausführungsbeispiels weist einen einteiligen Einstechabschnitt 13 auf, der auch während der Applikation, im Ausführungsbeispiel der Produktverabreichung, im Gewebe verbleibt. Das Stabilisierungselement 14 des ersten Ausführungsbeispiels entfällt. Der Einstechabschnitt 13 kann als herkömmliche Einstechnadel, insbesondere Einstechkanüle, gebildet sein, beispielsweise als Stahlnadel. Ein derartiger Einstechabschnitt 13 sollte möglichst flexibel sein, um im Gewebe möglichst wenig Irritation hervorzurufen. Er muss jedoch ein Mindestmaß an Biegesteifigkeit aufweisen, um in und vorzugsweise durch die Haut weiter in das Gewebe eindringen zu können. In einer bevorzugten Weiterentwicklung besteht der im Gewebe verbleibende Einstechabschnitt 13, der auch in der Weiterentwicklung ein Schneidelement für das Eindringen in das Gewebe bildet, zumindest teilweise aus einem Material, das durch den Kontakt mit dem Gewebe biegsamer wird. Geeignete Materialien sind insbesondere Polymere, die wenigstens einen Bestandteil der Körperflüssigkeit binden, mit der der Einstechabschnitt 13 im Gewebe in Kontakt kommt bzw. ist.

Bevorzugterweise ist dieser bindbare Bestandteil Wasser. Durch die Bindung wird das betreffende Material weicher, behält seine Integrität jedoch soweit bei, dass der durch den Einstechabschnitt 13 gebildete Strömungsquerschnitt für den Transport des Produkts durchgehend offen bleibt.

Der Insertionskopf des zweiten Ausführungsbeispiels weist einen gegenüber dem ersten Ausführungsbeispiel modifizierten Griff 15a auf. Der Griff 15a entspricht der Form nach dem Griff 15 des ersten Ausführungsbeispiels, ist jedoch nicht mehr mit einem Stabilisierungselement verbunden, sondern als reines Griffteil 15a gebildet. Der Griff 15a dient der Überführung der Einstecheinrichtung in die Einstechposition und gegebenenfalls auch wieder der Rückführung aus der Einstechposition in die Schutzposition. Der Griff 15a kann ferner wie der Griff 15 des ersten Ausführungsbeispiels als Griff für den gesamten Insertionskopf verwendet werden, um diesen auf dem Gewebe, insbesondere der menschlichen Haut, zu platzieren. Der Griff 15a kann ferner auch als Griff bei dem Lösen des Insertionskopfs von dem Gewebe dienen. Während der Applikation selbst ist der Griff 15a vom Rest des Insertionskopfs gelöst, so dass dieser auf der Gewebeoberfläche ein flaches Bauteil darstellt. Für das Lösen von der Gewebeoberfläche kann der Griff 15a wieder mit der Verbindungsstruktur 6 verrastet und als Handhabungshilfe für das Lösen verwendet werden. Der Griff 15a kann als Zusatzteil auch zum Lösen des Insertionskopfs des ersten Ausführungsbeispiel verwendet werden.

Im Übrigen entspricht der Insertionskopf des zweiten Ausführungsbeispiels dem des ersten, wobei lediglich der Vollständigkeit wegen darauf hingewiesen wird, dass die Verbindungsstruktur 6 wegen des Wegfalls des Stabilisierungselements 14 natürlich auch keinen Durchgang für das Stabilisierungselement 14 aufweisen muss. Der Durchgang 11 ist dementsprechend entfallen, könnte aber grundsätzlich bei entsprechender Abdichtung in der Verbindungsstruktur 6 verbleiben.

Die Fluidkammer 10 könnte insbesondere im zweiten Ausführungsbeispiel zu einem einfachen Strömungsquerschnitt umgeformt werden, der den Einstechabschnitt 13 kontinuierlich bis zum Durchgang 12 verlängert und in dem vorzugsweise eine Dichtscheibe angeordnet ist, um den Durchgang bzw. Anschluss 12 zur Umgebung abzudichten.

### Bezugszeichen:

- 1: Gehäuse
- 2: Unterseite
- 3: Aufnahme
- 4: Auflage, Führung, Stützabschnitt
- 5: Gelenkelement, Lagerzapfen
- 6: Verbindungsstruktur
- 7: flexibles Eindringelement, Leitungselement
- 8: Dichtelement
- 9: Halter
- 10: Fluidkammer
- 11: Durchgang
- 1 2: Anschluss, Durchgang
- 13: Einstechabschnitt, Einstechnadel
- 14: Stabilisierungselement
- 15: Griff
- 15a: Griff
- 16: Verbindungselement
- 17: -
- 18: Konnektor
- 19: Leitungselement, Infusatzuleitung
- 20: Verbindungsnadel
- 21: Verbindungselement

## Patentansprüche

1. Insertionskopf für medizinische oder pharmazeutische Anwendungen, der Insertionskopf umfassend ein Gehäuse (1) mit einer auf organischem Gewebe platzierbaren Unterseite (2) und eine Einstecheinrichtung (5-16), die einen mit dem Gehäuse schwenkbar verbundenen Einstechabschnitt (7, 14; 13) mit einer Spitze aufweist, wobei die Spitze in einer Einstechposition über die Unterseite (2) des Gehäuses vorragt, in einer Schutzposition, in der das Gehäuse die Spitze verdeckt, eine Aufnahme (3) des Gehäuses wenigstens die Spitze des Einstechabschnitts (7, 14; 13) aufnimmt, und in der Einstechposition der Einstechabschnitt eine relativ zu der Schutzposition gekippte Lage einnimmt, und wobei die Einstecheinrichtung eine mit dem Gehäuse verbundene Verbindungsstruktur (6) umfasst, von welcher der Einstechabschnitt abragt, und das Gehäuse und die Verbindungsstruktur miteinander ein Gelenk (5) bilden, in dem die Einstecheinrichtung (5-16) beweglich mit dem Gehäuse (1) verbunden ist,
**dadurch gekennzeichnet, dass**
die Einstecheinrichtung für eine Überführung von der Schutzposition in die Einstechposition und/oder von der Einstechposition in die Schutzposition ein mit der Verbindungsstruktur lösbar verbundenes Griffteil (15a; 15) umfasst, wobei das Griffteil im verbundenen Zustand von der Verbindungsstruktur abragt, und
das Griffteil zwei gegen dessen rückstellende Elastizität gegeneinander drückbare äussere Endbereiche mit je einem Verbindungselement (16) aufweist, wobei die beiden Verbindungselemente von der Innenseite der äusseren Endbereiche radial einwärts vorragen und je ein hakenförmiges Rastelement bilden, das mit einem entsprechenden Gegenelement in einer Ausnehmung der Verbindungsstruktur lösbar verrastet ist.

2. Insertionskopf nach dem vorhergehenden Anspruch, **dadurch gekennzeichnet, dass** die Einstecheinrichtung (5-16) mit dem Gehäuse (1) aus der Schutzposition in die Einstechposition bewegbar permanent verbunden ist.

3. Insertionskopf nach einem der vorhergehenden Ansprüche, **dadurch gekennzeichnet, dass** die Einstecheinrichtung (5-16) und das Gehäuse (1) miteinander ein Gelenk (5) mit einer quer zu dem Einstechabschnitt (7, 14; 13) weisenden Gelenkachse bilden, um die die Einstecheinrichtung (5-16) relativ zu dem Gehäuse (1) drehbar ist.

4. Insertionskopf nach einem der vorhergehenden Ansprüche, **dadurch gekennzeichnet, dass** das Gehäuse (1) die Einstecheinrichtung (5-16) auch in einer vorgegebenen weiteren Einstechposition hält, wobei der Einstechabschnitt (7, 14; 13) in der einen der Einstechpositionen zu der Unterseite (2) eine andere Neigung als in der anderen aufweist.

5. Insertionskopf nach einem der vorhergehenden Ansprüche, **dadurch gekennzeichnet, dass** das Gehäuse (1) die Einstecheinrichtung (5-16) form- und/oder kraftschlüssig, vorzugsweise in einer Rastverbindung, in wenigstens einer der Positionen hält.

6. Insertionskopf nach einem der vorhergehenden Ansprüche, **dadurch gekennzeichnet, dass** die Aufnahme (3) zu einer Außenseite (2) des Gehäuses (1) offen ist.

7. Insertionskopf nach dem vorhergehenden Anspruch, **dadurch gekennzeichnet, dass** die Aufnahme (3) zu der Unterseite (2) offen ist.

8. Insertionskopf nach einem der vorhergehenden Ansprüche, **dadurch gekennzeichnet, dass** die Einstecheinrichtung (5-16) eine Verbindungsstruktur (6) umfasst, die mit dem Gehäuse (1) gelenkig verbunden ist und von der der Einstechabschnitt (7, 14; 13) abragt, und dass die Aufnahme (3) sich zu der Verbindungsstruktur (6) öffnet.

9. Insertionskopf nach einem der vorhergehenden Ansprüche, **dadurch gekennzeichnet, dass** der Einstechabschnitt (7, 14; 13) ein Leitungselement (7) für ein Fluid umfasst oder ist.

10. Insertionskopf nach dem vorhergehenden Anspruch, **dadurch gekennzeichnet, dass** die Einstecheinrichtung (5-16) eine mit dem Leitungselement (7) verbundene Fluidkammer (10) mit einem Fluidanschluss (12) und eine Dichtung (8) für den Fluidanschluss (12) umfasst.

11. Insertionskopf nach einem der vorhergehenden Ansprüche, **dadurch gekennzeichnet, dass** die Verbindungsstruktur (6) einen Anschluss (12) für ein Fluid oder Signale aufweist.

12. Insertionskopf nach einem der vorhergehenden Ansprüche, **dadurch gekennzeichnet, dass** das Gehäuse (1) und die Verbindungsstruktur (6) miteinander ein Gelenk (5) bilden, in dem die Einstecheinrichtung (5-16) beweglich zwischen Rastpositionen, in denen die Verbindungsstruktur (6) und das Gehäuse (1) verrasten, mit dem Gehäuse verbunden ist.

13. Insertionskopf nach einem der vorhergehenden Ansprüche, **dadurch gekennzeichnet, dass** das Gehäuse (1) eine Auflage (4) mit einer von der Unterseite (2) abgewandten Auflagefläche bildet, auf der das Griffteil (15a; 15) aufliegt, wenn die Einstecheinrichtung (5-16) die Schutzposition einnimmt.

14. Insertionskopf nach einem der vorhergehenden Ansprüche, **dadurch gekennzeichnet, dass** der Einstechabschnitt (7, 14) ein flexibles Eindringelement (7) und ein Stabilisierungselement (14) für das Eindringelement (7) umfasst und dass das Stabilisierungselement (14) lösbar mit der Verbindungsstruktur (6) verbunden ist.

15. Insertionskopf nach dem vorhergehenden Anspruch, **dadurch gekennzeichnet, dass** das Griffteil (15) das Stabilisierungselement (14) an einem proximalen Ende hält und das Gehäuse (1) eine Auflage (4) mit einer von der Unterseite (2) abgewandten Auflagefläche bildet, auf der das Griffteil (15) aufliegt, wenn die Einstecheinrichtung (5-16) die Schutzposition einnimmt.

16. Insertionskopf nach einem der zwei vorhergehenden Ansprüche, **dadurch gekennzeichnet, dass** das Stabilisierungselement (14) mittels einer Rastverbindung an der Verbindungsstruktur (6) angebracht ist.

17. Insertionskopf nach einem der vorhergehenden Ansprüche, **dadurch gekennzeichnet, dass** die Einstecheinrichtung durch ein flexibles Eindringelement (7), beispielsweise in Form einer flexiblen Kanüle mit einem inneren Strömungsquerschnitt, und ein biegesteifes Stabilisierungselement (14), in Form einer Einstechnadel, gebildet wird, wobei das Stabilisierungselement die Verbindungsstruktur und das davon abragende Eindringelement durchragt, die freie Spitze des Stabilisierungselements über das freie Ende des flexiblen Eindringelements vorsteht, und sich das Eindringelement mit einer leichten Spannung an die Mantelaussenfläche des Stabilisierungselements anschmiegt.

18. Insertionskopf nach dem vorhergehenden Anspruch, **dadurch gekennzeichnet, dass** das Griffteil (15) das Stabilisierungselement (14) an einem proximalen Ende hält, wobei nach einem Lösen des Griffteils von der Verbindungstruktur (6) das Stabilisierungselement mit dem Griffteil aus dem Eindringelement (7) herausgezogen werden kann.

19. Insertionskopf nach einem der Ansprüche 1 bis 16, **dadurch gekennzeichnet, dass** eine Einstechnadel (13), die während der Applikation im Gewebe verbleibt, den Einstechabschnitt (13) bildet.

20. Insertionskopf nach einem der Ansprüche 1 bis 17, **dadurch gekennzeichnet, dass** der Einstechabschnitt (13) eine Einstechnadel (13) ist oder umfasst, die während der Applikation im Gewebe verbleibt, und dass die Biegsamkeit der Einstechnadel (13) bei einem Kontakt mit dem Gewebe zunimmt.

21. Insertionskopf nach dem vorhergehenden Anspruch, **dadurch gekennzeichnet, dass** die Einstechnadel (13) zumindest teilweise aus einem Polymermaterial besteht, das wenigstens einen Bestandteil einer Körperflüssigkeit bindet und dass die Biegsamkeit der Einstechnadel (13) durch die Bindung zunimmt.

22. Insertionskopf nach einem der vorhergehenden Ansprüche, **dadurch gekennzeichnet, dass** das Griffteil (15a; 15) für die Handhabung des Insertionskopf bei dem Platzieren auf oder einem Lösen von dem Gewebe von dem Gehäuse (1) abragt.

23. Insertionskopf nach einem der vorhergehenden Ansprüche, **dadurch gekennzeichnet, dass** der Insertionskopf einen an die Einstecheinrichtung (5-16) anschließbaren Konnektor (18) mit einem Leitungselement (19) für ein Fluid oder Signale umfasst.

24. Insertionskopf nach dem vorhergehenden Anspruch, **dadurch gekennzeichnet, dass** der Konnektor (18) mechanisch mit dem Gehäuse (1) oder der Verbindungsstruktur (6) verbindbar ist.

25. Insertionskopf nach einem der zwei vorhergehenden Ansprüche in Kombination mit einem der Ansprüche 13 oder 15, **dadurch gekennzeichnet, dass** die Auflage (4) eine Führung für den Konnektor (18) bildet, wenn der Konnektor (18) mit der Einstecheinrichtung (5-16) verbunden wird.

## Claims

1. An insertion head for medical or pharmaceutical applications, the insertion head comprising a housing (1) having an underside (2) which can be positioned on organic tissue and a puncturing device (5-16) which comprises a puncturing section (7, 14; 13) which is connected to the housing in a pivotal manner and which has a tip, wherein in a puncturing position, the tip protrudes beyond the underside (2) of the housing, in a protected position in which the housing covers the tip, a recess (3) accommodates at least the tip of the puncturing section (7, 14; 13) and in the puncturing position, the puncturing section adopts a position which is tilted relative to the protected position, and in which the puncturing device comprises a connecting structure (6) which is connected to the housing, from which the puncturing section protrudes, and the housing and the connecting structure together form an joint (5) in which the puncturing device (5-16) is movably connected to the housing (1),
**characterized in that**
for a transfer from the protected position into the puncturing position and/or from the puncturing position into the protected position, the puncturing device comprises a handle part (15a; 15) which is releasably connected to the connecting structure, wherein in the connected condition, the handle protrudes from the connecting structure, and the handle part comprises two outer end regions which can be pressed towards each other against their restoring elasticity, each having a connecting element (16), wherein the two connecting elements protrude radially inwards from the inner side of the outer end regions and each forms a hook-shaped latching element which is latched with a corresponding counter-element in a recess of the connecting structure.

2. The insertion head as claimed in the preceding claim, **characterized in that** the puncturing device (5- 16) is permanently connected to the housing (1) in a manner such that it is movable from the protected position into the puncturing position.

3. The insertion head as claimed in one of the preceding claims, **characterized in that** the puncturing device (5-16) and the housing (1) together form a joint (5) with a joint axis orientated transversely to the puncturing section (7, 14; 13) about which the puncturing device (5-16) is rotatable relative to the housing (1).

4. The insertion head as claimed in one of the preceding claims, **characterized in that** the housing (1) also holds the puncturing device (5-16) in a predetermined further puncturing position, wherein in one of the puncturing positions the puncturing section (7, 14; 13) is at an inclination with respect to the underside (2) which differs from that in the other puncturing position.

5. The insertion head as claimed in one of the preceding claims, **characterized in that** the housing (1) retains the puncturing device (5-16) interlocking and/or force-locking, preferably in a latching connection, in at least one of the positions.

6. The insertion head as claimed in one of the preceding claims, **characterized in that** the recess (3) is open towards an outer side (2) of the housing (1).

7. The insertion head as claimed in the preceding claim, **characterized in that** the recess (3) is open towards the underside (2).

8. The insertion head as claimed in one of the three preceding claims, **characterized in that** the puncturing device (5-16) comprises a connecting structure (6) which is connected to the housing (1) in an articulated manner and from which the puncturing section (7, 14; 13) protrudes, and **in that** the recess (3) opens towards the connecting structure (6).

9. The insertion head as claimed in one of the preceding claims, **characterized in that** the puncturing section (7, 14; 13) comprises or is a line element (7) for a fluid.

10. The insertion head as claimed in the preceding claim, **characterized in that** the puncturing device (5- 16) comprises a fluid chamber (10) which is connected to the line element (7) and has a fluid connection (12) and a seal (8) for the fluid connection (12).

11. The insertion head as claimed in the preceding claim, **characterized in that** the connecting structure (6) is provided with a connection (12) for a fluid or signals.

12. The insertion head as claimed in one of the preceding claims, **characterized in that** the housing (1) and the connecting structure (6) together form a joint (5) in which the puncturing device (5-16) is movably connected to the housing between latching positions in which the connecting structure (6) and the housing (1) are latched.

13. The insertion head as claimed in the preceding claim, **characterized in that** the housing (1) forms a support (4) with a support surface facing away from the underside (2), on which the handle part (15a; 15) rests when the puncturing device (5-16) adopts the protected position.

14. The insertion head as claimed in one of the preceding claims, **characterized in that** the puncturing section (7, 14) comprises a flexible penetrating element (7) and a stabilizing element (14) for the penetrating element (7), and **in that** the stabilizing element (14) is releasably connected to the connecting structure (6).

15. The insertion head as claimed in the preceding claim, **characterized in that** the handle part (15) retains the stabilizing element (14) at a proximal end and the housing (1) forms a support (4) with a support surface facing away from the underside (2) on which the handle part (15) rests when the puncturing device (5-16) adopts the protected position.

16. The insertion head as claimed in one of the two preceding claims, **characterized in that** the stabilizing element (14) is attached to the connecting structure (6) by means of a latching connection.

17. The insertion head as claimed in one of the preceding claims, **characterized in that** the puncturing device is formed by a flexible penetrating element (7), for example in the form of a flexible cannula with an inner cross section of flow, and a rigid stabilizing element (14) in the form of a penetrating needle, wherein the stabilizing element passes through the connecting structure and the penetrating element protruding therefrom, the free tip of the stabilizing element protrudes beyond the free end of the flexible penetrating element, and the penetrating element fits snugly onto the peripheral outer surface of the stabilizing element with a slight tension.

18. The insertion head as claimed in the preceding claim, **characterized in that** the handle part (15) retains the stabilizing element (14) at a proximal end, wherein after releasing the handle part from the connecting structure (6), the stabilizing element with the handle part can be withdrawn from the penetrating element (7).

19. The insertion head as claimed in one of claims 1 to 16, **characterized in that** a puncturing needle (13), which remains in the tissue during the application, forms the puncturing section (13).

20. The insertion head as claimed in one of claims 1 to 17, **characterized in that** the puncturing section (13) is or comprises a puncturing needle (13), which remains in the tissue during the application, and **in that** the flexibility of the puncturing needle (13) increases upon contact with the tissue.

21. The insertion head as claimed in the preceding claim, **characterized in that** the puncturing needle (13) at least partially consists of a polymer material which binds at least one component of a body fluid, and **in that** the flexibility of the puncturing needle (13) increases as a result of the binding.

22. The insertion head as claimed in one of the preceding claims, **characterized in that** the handle part (15a; 15) protrudes from the housing (1) for handling the insertion head when positioning on the tissue or releasing from the tissue.

23. The insertion head as claimed in one of the preceding claims, **characterized in that** the insertion head comprises a connector (18) having a line element (19) for a fluid or signals which can be attached to the puncturing device (5-16).

24. The insertion head as claimed in the preceding claim, **characterized in that** the connector (18) can be mechanically connected to the housing (1) or to the connecting structure (6).

25. The insertion head as claimed in one of the two preceding claims in combination with one of claims 13 or 15, **characterized in that** the support (4) forms a guide for the connector (18) when the connector (18) is connected to the puncturing device (5-16).

## Revendications

1. Tête d'insertion pour applications médicales ou pharmaceutiques, la tête d'insertion comprenant un boîtier (1) doté d'une face inférieure (2) pouvant être placée sur du tissu organique et un dispositif de piquage (5-16) qui présente une section de piquage (7, 14 ; 13) connectée de manière pivotable au boîtier et dotée d'une pointe, la pointe dépassant, dans une position de piquage, au-delà de la face inférieure (2) du boîtier, sachant que, dans une position de protection dans laquelle le boîtier couvre la pointe, un support (3) du boîtier reçoit au moins la pointe de la section de piquage (7, 14 ; 13) et que, dans la position de piquage, la section de piquage prend une position basculée par rapport à la position de protection, et le dispositif de piquage comprenant une structure de connexion (6) connectée au boîtier et de laquelle dépasse la section de piquage, et le boîtier et la structure de connexion constituant ensemble une articulation (5) dans laquelle le dispositif de piquage (5-16) est connecté de manière mobile au boîtier (1),
**caractérisée en ce que**
le dispositif de piquage, pour un transfert de la position de protection vers la position de piquage et/ou de la position de piquage vers la position de protection, comprend un élément de préhension (15a ; 15) connecté de manière dissociable à la structure de connexion, l'élément de préhension dépassant, en état connecté, de la structure de connexion, et
l'élément de préhension présente deux parties terminales extérieures pouvant être comprimées l'une contre l'autre à l'encontre de son élasticité de rappel et comportant chacune un élément de connexion (16), les deux éléments de connexion dépassant radialement vers l'intérieur depuis la face interne des parties terminales extérieures et formant chacune un élément d'enclenchement en forme de crochet qui s'enclenche de manière dissociable dans un contre-élément correspondant dans un évidement de la structure de connexion.

2. Tête d'insertion selon la revendication précédente, **caractérisée en ce que** le dispositif de piquage (5-16) est connecté de manière permanente au boîtier (1) en pouvant passer de la position de protection à la position de piquage.

3. Tête d'insertion selon une des revendications précédentes, **caractérisée en ce que** le dispositif de piquage (5-16) et le boîtier (1) constituent ensemble une articulation (5) dotée d'un axe d'articulation orienté transversalement à la section de piquage (7, 14 ; 13) et autour duquel le dispositif de piquage (5-16) peut tourner par rapport au boîtier (1).

4. Tête d'insertion selon une des revendications précédentes, **caractérisée en ce que** le boîtier (1) maintient le dispositif de piquage (5-16) également dans une autre position de piquage prédéfinie, la section de piquage (7, 14 ; 13) présentant, dans une des positions de piquage par rapport à la face inférieure (2), une inclinaison autre que dans l'autre position.

5. Tête d'insertion selon une des revendications précédentes, **caractérisée en ce que** le boîtier (1) maintient le dispositif de piquage (5-16) en correspondance géométrique et/ou mécanique, de préférence dans une connexion à enclenchement, dans au moins une des positions.

6. Tête d'insertion selon une des revendications précédentes, **caractérisée en ce que** le support (3) est ouvert vers une face extérieure (2) du boîtier (1).

7. Tête d'insertion selon la revendication précédente, **caractérisée en ce que** le support (3) est ouvert vers la face inférieure (2).

8. Tête d'insertion selon une des revendications précédentes, **caractérisée en ce que** le dispositif de piquage (5-16) comprend une structure de connexion (6) qui est connectée de manière articulée au boîtier (1) et dépasse de la section de piquage (7, 14 ; 13) et que le support (3) s'ouvre en direction de la structure de connexion (6).

9. Tête d'insertion selon une des revendications précédentes, **caractérisée en ce que** la section de piquage (7, 14 ; 13) comprend ou est un élément conducteur (7) pour un fluide.

10. Tête d'insertion selon une des revendications précédentes, **caractérisée en ce que** le dispositif de piquage (5-16) comprend une chambre à fluide (10) connectée à l'élément conducteur (7) et dotée d'un raccord de fluide (12) et comprend un joint (8) pour le raccord de fluide (12).

11. Tête d'insertion selon une des revendications précédentes, **caractérisée en ce que** la structure de connexion (6) présente un raccord (12) pour un fluide ou des signaux.

12. Tête d'insertion selon une des revendications précédentes, **caractérisée en ce que** le boîtier (1) et la structure de connexion (6) constituent ensemble une articulation (5) dans laquelle le dispositif de piquage (5-16) est connecté au boîtier de manière mobile entre des positions d'enclenchement dans lesquelles la structure de connexion (6) et le boîtier (1) s'enclenchent.

13. Tête d'insertion selon une des revendications précédentes, **caractérisée en ce que** le boîtier (1) constitue un support (4) avec une surface d'appui détournée de la face inférieure (2) et sur laquelle repose la pièce de préhension (15a ; 15) lorsque le dispositif de piquage (5-16) prend la position de protection.

14. Tête d'insertion selon une des revendications précédentes, **caractérisée en ce que** la section de piquage (7, 14) comprend un élément de pénétration flexible (7) et un élément de stabilisation (14) pour l'élément de pénétration (7) et que l'élément de stabilisation (14) est connecté de manière dissociable à la structure de connexion (6).

15. Tête d'insertion selon une des revendications précédentes, **caractérisée en ce que** l'élément de préhension (15) maintient l'élément de stabilisation (14) à une extrémité proximale et que le boîtier (1) constitue un support (4) avec une surface d'appui détournée de la face inférieure (2) et sur laquelle repose la pièce de préhension (15) lorsque le dispositif de piquage (5-16) prend la position de protection.

16. Tête d'insertion selon une des revendications précédentes, **caractérisée en ce que** l'élément de stabilisation (14) est installé au moyen d'une connexion à enclenchement au niveau de la structure de connexion (6).

17. Tête d'insertion selon une des revendications précédentes, **caractérisée en ce que** le dispositif de piquage est constitué par un élément de pénétration flexible (7), par exemple sous la forme d'une canule flexible dotée d'une surface transversale intérieure d'écoulement, et d'un élément de stabilisation rigide à la flexion (14) sous forme d'une aiguille de piquage, l'élément de stabilisation traversant la structure de connexion et l'élément de pénétration en dépassant, la pointe libre sortie de l'élément de stabilisation dépassant au-delà de l'extrémité libre de l'élément de pénétration flexible et l'élément de pénétration épousant avec une légère tension la surface extérieure d'enveloppe de l'élément de stabilisation.

18. Tête d'insertion selon la revendication précédente, **caractérisée en ce que** l'élément de préhension (15) maintient l'élément de stabilisation (14) à une extrémité proximale, sachant que, après un détachement de l'élément de préhension de la structure de connexion (6), l'élément de stabilisation peut être sorti avec l'élément de préhension de l'élément de pénétration (7).

19. Tête d'insertion selon une des revendications 1 à 16, **caractérisée en ce qu'**une aiguille de piquage (13) qui reste dans le tissu pendant l'application constitue la section de piquage (13).

20. Tête d'insertion selon une des revendications 1 à 17, **caractérisée en ce que** la section de piquage (13) est ou comprend une aiguille de piquage (13) qui reste dans le tissu pendant l'application et que la souplesse de l'aiguille de piquage (13) augmente lors d'un contact avec le tissu.

21. Tête d'insertion selon la revendication précédente, **caractérisée en ce que** l'aiguille de piquage (13) est composée au moins partiellement d'un matériau polymérique qui lie au moins une composante d'un liquide physiologique et que la souplesse de l'aiguille de piquage (13) augmente du fait de cette liaison.

22. Tête d'insertion selon une des revendications précédentes, **caractérisée en ce que** l'élément de préhension (15a ; 15), pour le maniement de la tête d'insertion lors du placement sur ou d'un détachement du tissu, dépasse du boîtier (1).

23. Tête d'insertion selon une des revendications précédentes, **caractérisée en ce que** la tête d'insertion comprend un connecteur (18) pouvant être raccordé au dispositif de piquage (5-16) et doté d'un élément conducteur (19) pour un fluide ou des signaux.

24. Tête d'insertion selon la revendication précédente, **caractérisée en ce que** le connecteur (18) est connectable mécaniquement au boîtier (1) ou à la structure de connexion (6).

25. Tête d'insertion selon une des deux revendications précédentes en combinaison avec une des revendications 13 ou 15, **caractérisée en ce que** le support (4) constitue un guide pour le connecteur (18) lorsque le connecteur (18) est connecté au dispositif de piquage (5-16).
